(19) Europäisches Patentamt
European Patent Office
Office européen des brevets

(11) Publication number: 0 221 996 B1

(12) **EUROPEAN PATENT SPECIFICATION**

(45) Date of publication of patent specification: **29.08.90**

(51) Int. Cl.$^5$: **C 07 D 487/14,** A 61 K 31/52 // (C07D487/14, 239:00, 235:00)

(21) Application number: **86903777.0**

(22) Date of filing: **12.05.86**

(86) International application number: **PCT/US86/01004**

(87) International publication number: **WO 86/06724 20.11.86 Gazette 86/25**

(54) **SUBSTITUTED 2,3-DIHYDRO-6-HYDROXY-PYRIMIDO 2,1-fr PURINE-4,8(1H,9H)-DIONES.**

(30) Priority: **13.05.85 US 732994**

(43) Date of publication of application: **20.05.87 Bulletin 87/21**

(45) Publication of the grant of the patent: **29.08.90 Bulletin 90/35**

(84) Designated Contracting States: **AT BE CH DE FR GB IT LI LU NL SE**

(56) References cited: **EP-A-0 107 165**

(73) Proprietor: **SCHERING CORPORATION**
**2000 Galloping Hill Road**
**Kenilworth New Jersey 07033 (US)**

(72) Inventor: **SOLOMON, Daniel Mordecai**
**9 Marshall Drive**
**Edison, NJ 08817 (US)**
Inventor: **KAMINSKI, James Joseph**
**3 Fleming Court**
**Long Valley, NJ 07853 (US)**
Inventor: **CONN, David Jeffry**
**502 New Center Road**
**Somerville, NJ 08876 (US)**

(74) Representative: **von Kreisler, Alek, Dipl.-Chem. et al**
**Deichmannhaus am Hauptbahnhof**
**D-5000 Köln 1 (DE)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Courier Press, Leamington Spa, England.

EP 0 221 996 B1

## Description

The present invention relates to substituted 2,3-dihydro-6-hydroxy-pyrimido[2,1-f]purine-4,8(1*H*,9*H*)-diones and tautomers thereof. These compounds are useful as anti-inflammatory agents for treating inflammatory conditions such as arthritis, spondylitis, tendonitis, eczema and psoriasis in mammals. Also these compounds are useful as anti-allergy agents for treating allergy-caused diseases.

The invention provides compounds having the structural formula I

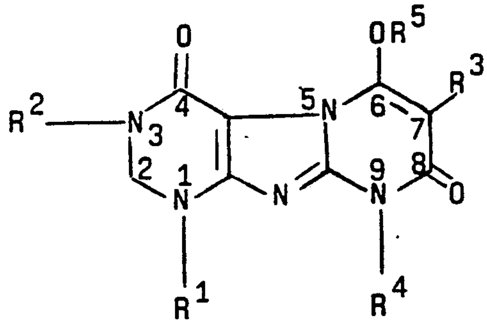

I

including the tautomers and pharmaceutically acceptable salts, wherein

$R^1$ and $R^2$ are independently selected from hydrogen, cycloalkyl having from 3 to 8 carbon atoms, phenyl, substituted phenyl, lower alkyl, and lower alkyl substituted with cycloalkyl having from 3 to 8 carbon atoms, with phenyl, with thienyl or with substituted phenyl;

$R^3$ is selected from hydrogen, formyl, cycloalkyl having from 3 to 8 carbon atoms, alkenyl having from 2 to 8 carbon atoms, alkenyl having from 3 to 8 carbon atoms and substituted with up to 6 fluorines, alkynyl having from 3 to 8 carbon atoms, cycloalkenyl having from 5 to 8 carbon atoms, acyloxyalkyl having from 2 to 12 carbon atoms, phenyl, substituted phenyl, lower alkyl, X—$R^6$, -alkyl—Y—$C_pH_{2p+1}$ wherein the alkyl portion has 1 to 6 carbon atoms, —$(CH_2)_nCONR^7R^8$, —$(CH_2)_mCO$—$OR^9$, and lower alkyl substituted with hydroxy, with sulfhydryl, with cyano, with amino, with halo, with cycloalkyl having from 3 to 8 carbon atoms, with phenyl, with thienyl or with substituted phenyl;

wherein X is 0, NH or S;

$R^6$ is phenyl, substituted phenyl, lower alkyl, or lower alkyl substituted with cycloalkyl having from 3 to 8 carbon atoms or with phenyl;

p is an integer from 0 to 4;

Y represents CO, O, S, $S^+$—$O^-$, $SO_2$ or $=NC_rH_{2r+1}$ wherein r is an integer from 0 to 4, with the proviso that p is an integer from 1 to 4 when Y is $S^+$—$O^-$ or $SO_2$;

$R^7$ and $R^8$ are independently hydrogen or lower alkyl;

n is an integer from 0 to 6;

$R^9$ is a hydrogen or lower alkyl;

and m is an integer from 0 to 6;

$R^4$ is selected from hydrogen, phenyl, thienyl, pyridyl, substituted phenyl, lower alkyl, and lower alkyl substituted with cycloalkyl having from 3 to 8 carbon atoms, with phenyl, with thienyl or with substituted phenyl; and

$R^5$ is selected from hydrogen and alkyl having from 1 to 4 carbon atoms;

the term "substituted" in relation to phenyl, pyridyl and thienyl groups means such groups substituted with 1 to 3 substituents independently selected from halogen, trifluoromethyl, $CONH_2$, —$CO_2H$, hydroxy, —$S(O)_aR^{10}$ wherein $R^{10}$ is lower alkyl and a is 0, 1 or 2, —$OR^{11}$ wherein $R^{11}$ is lower alkyl, or $COR^{12}$ wherein $R^{12}$ is lower alkyl or alkoxy having from 1 to 6 carbon atoms;

and the term "lower" in relation to alkyl groups indicates such groups having from 1 to 6 carbon atoms.

The pharmaceutically acceptable salts are normally formed by replacement of a hydrogen atom as $R^9$ or $R^5$ with one equivalent of a pharmaceutically acceptable metal or amine cation. Compounds wherein X is NH and/or Y is $=NC_rH_{2r+1}$ can be prepared in the form of their pharmaceutically acceptable acid addition salts.

When utilized herein and in the appended claims the listed terms below, unless specified otherwise, are defined as follows:

"halogen" means fluorine, chlorine, bromine and iodine; and

"lower alkyl" means straight or branched chain alkyls having 1 to 6 carbons, e.g. methyl, ethyl, propyl, isopropyl, butyl, t-butyl, 2,2-dimethylpropyl, pentyl, hexyl.

"Pharmaceutically acceptable metal and amine cations" includes in particular lithium, sodium, potassium, magnesium, calcium, aluminum, zinc, iron, copper, gold, ammonium, ethylenediamine, mono-, di- and tri-ethanolamine, ethyldiethanolamine, *n*-butylethanolamine, 2-amino-2-methyl-1-propanol, tris-(hydroxymethyl)-aminomethane, lysine, galactamine, N-methyl-glucosamine.

"Pharmaceutically acceptable acid addition salts" includes in particular those formed with hydrochloric, hydrobromic, sulfuric, phosphoric, methanèsulfonic, citric, tartaric, maleic or fumaric acid.

Many compounds of formula I (including their pharmaceutically acceptable salts) exist as solvates, especially hydrates. All such solvates are included in the embodiments of the present invention.

Compounds of the formula I wherein $R^5$ is hydrogen may exist in tautomeric forms:

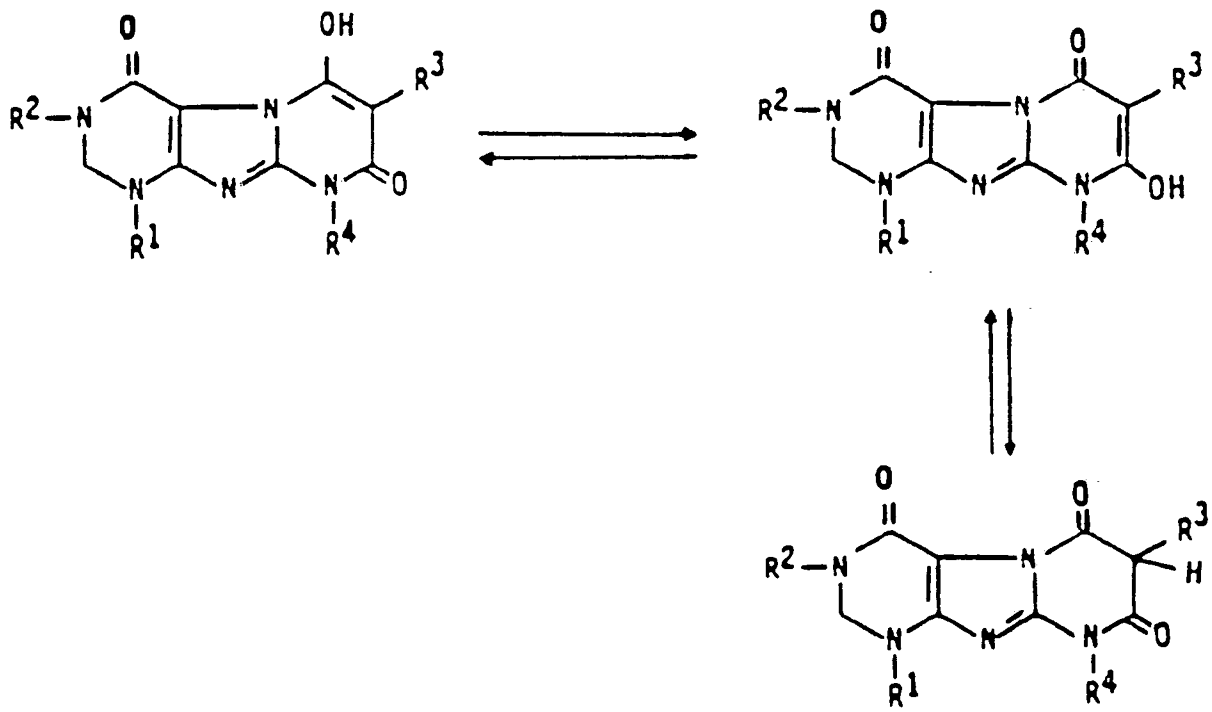

Such tautomeric forms are equivalent for purposes of the invention.

Preferred embodiments of the present invention relate to compounds having the structural formula I and the pharmaceutically acceptable salts thereof when $R^5$ is hydrogen, wherein

$R^1$ and R 05 are independently selected from alkyl having from 1 to 4 carbon atoms;

$R^3$ is hydrogen, alkenyl, having from 2 to 8 carbon atoms which may be substituted with up to 6 fluorines, alkynyl having from 3 to 8 carbon atoms, cycloalkenyl having from 5 to 8 carbon atoms, lower alkyl, or lower alkyl substituted with phenyl;

$R^4$ is lower alkyl which is substituted with phenyl, with thienyl or with substituted phenyl; and $R^5$ is hydrogen.

More preferred values for $R^1$ and $R^2$ are alkyl having 1 to 3 carbon atoms. Most preferably $R^1$ and $R^2$ are methyl.

More preferred values for $R^3$ are hydrogen, alkenyl having from 3 to 8 carbon atoms which may be substituted with up to 6 fluorines, alkynyl having from 3 to 8 carbon atoms, cycloalkenyl having from 5 to 8 carbon atoms, lower alkyl, and lower alkyl substituted with phenyl. Most preferably $R^3$ is hydrogen, methyl, n-propyl, 2-propynyl, allyl, trans-2-butenyl, 2-cyclohexenyl, $—CH_2CH=C(CH_3)_2$ (prenyl), $—CH_2CH=C(CF_3)CH_3$, $—CH_2CH=C(CF_3)_2$ or benzyl.

More preferred values for $R^4$ are benzyl, 2-thienylmethyl and substituted benzyl. Most preferably $R^4$ is 2-thienylmethyl, benzyl or p-fluoro-benzyl.

Pharmaceutically acceptable salts of compounds of formula I wherein $R^5$ is hydrogen preferably contain a metal cation, most preferably the sodium cation.

Particularly preferred compounds having structural formula I are as follows:

9-benzyl-2,3-dihydro-1,3-dimethyl-6-hydroxy-7-propyl-pyrimido[2,1-f]purine-4,8(1*H*,9*H*)-dione, m.p. 177—178.5°C;

2,3-dihydro-1,3-dimethyl-9-(4-fluorobenzyl)-6-hydroxy-7-propyl-pyrimido[2,1-f]purine-4,8(1*H*,9*H*)-dione, m.p. 139—140.5°C (dec);

9-benzyl-2,3-dihydro-1,3-dimethyl-6-hydroxy-7-(3-methyl-2-butenyl)-pyrimido[2,1-f]purine-4,8(1*H*,9*H*)-dione, m.p. 153—154.5°C (1/3 hydrate);

9-benzyl-2,3-dihydro-1,3-dimethyl-6-hydroxy--pyrimido[2,1-f]purine-4,8(1*H*,9*H*)-dione, m.p. 176—182°C;

2,3-dihydro-1,3-dimethyl-9-(4-fluorobenzyl)-6-hydroxy-7-(3-methyl-2-butenyl)-pyrimido[2,1-f]purine-4,8(1*H*,9*H*)-dione, m.p. 188—188.5°C;

9-benzyl-2,3-dihydro-1,3-dimethyl-6-hydroxy-7-methyl-pyrimido[2,1-f]purine-4,8(1*H*,9*H*)-dione, m.p. 222—224°C;

2,3-dihydro-1,3-dimethyl-9-(4-fluorobenzyl)-6-hydroxy-pyrimido[2,1-f]purine-4,8(1*H*,9*H*)-dione, m.p. 287—290°C (dec);

7,9-dibenzyl-2,3-dihydro-1,3-dimethyl-6-hydroxy-pyrimido[2,1-f]purine-4,8(1*H*,9*H*)-dione, m.p. 176—179°C;

9-benzyl-2,3-dihydro-1,3-dimethyl-7-formyl-6-hydroxy-pyrimido[2,1-f]purine-4,8(1*H*,9*H*)-dione, m.p. 325—326°C (dec);

2,3-dihydro-1,3-dimethyl-9-(4-fluorobenzyl)-6-hydroxy-7-2-propenyl-pyrimido[2,1-f]purine-4,8(1*H*,9*H*)-dione, m.p. 131.5—133°C (dec);

9-benzyl-2,3-dihydro-1,3-dimethyl-7-(2-propynyl)-6-hydroxy-pyrimido[2,1-f]purine-4,8(1*H*,9*H*)-dione, m.p. 163—164°C;

9-benzyl-7-(trans-2-butenyl)-2,3-dihydro-1,3-dimethyl-6-hydroxy-pyrimido[2,1-f]purine-4,8(1*H*,9*H*)-dione, m.p. 180—183°C;

9-benzyl-7-(3-cyclohexenyl)-2,3-dihydro-1,3-dimethyl-6-hydroxy-pyrimido[2,1-f]purine-4,8(1*H*,9*H*)-dione, m.p. 208—210°C;

9-benzyl-2,3-dihydro-1,3-dimethyl-7-ethoxycarbonylmethyl-6-hydroxy-pyrimido[2,1-f]purine-4,8(1*H*,9*H*)-dione, m.p. 148—150°C;

2,3-dihydro-1,3-di-(n-butyl)-6-hydroxy-7-methyl-9-(2-phenylethyl)-pyrimido[2,1-f]purine-4,8(1*H*,9*H*)-dione;

2,3-dihydro-1,3-dimethyl-9-(2-thienylmethyl)-6-hydroxy-7-propyl-pyrimido[2,1-f]purine-4,8(1*H*,9*H*)-dione, m.p. 186—188°C; and

2,3-dihydro-1,3-dimethyl-9-(4-methoxybenzyl)-6-hydroxy-7-(3-methyl-2-butenyl)-pyrimido[2,1-f]-purine-4,8(1*H*,9*H*)-dione, m.p. 157—159°C.

The above compounds are also preferred in the form of their sodium salts. In the above list the compounds are named for convenience as their 6-hydroxy-8-one tautomers, but the equivalent 8-hydroxy-6-one tautomers are equally covered.

The following reaction scheme illustrates a preferred process for the preparation of compounds of the present invention:

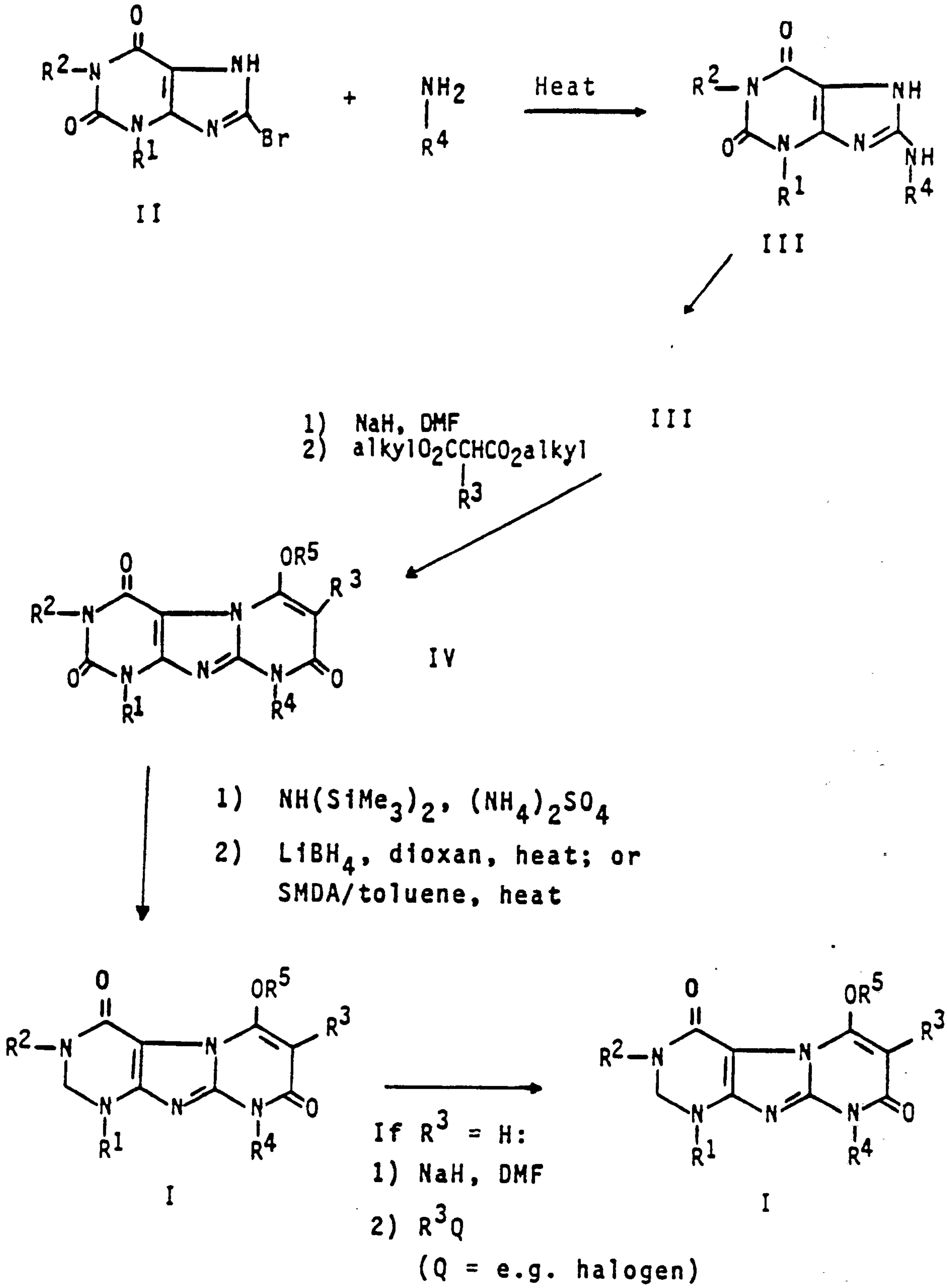


wherein $R^1$, $R^2$, $R^3$, $R^4$ and $R^5$ are as defined above. Some groups, especially in $R^3$, may be sensitive to the step of reduction, and compounds containing such $R^3$ groups are better prepared by reduction of compounds of the formula IV wherein $R^3$ is hydrogen followed by introduction of the group $R^3$ under basic alkylation conditions.

The invention therefore provides a process for the preparation of compounds of the formula I hereinabove defined which comprises reducing a compound of the formula IV

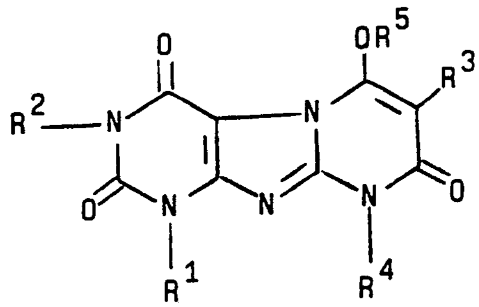

IV

or a silylated derivative thereof, wherein $R^1$, $R^2$, $R^3$, $R^4$ and $R^5$ are as defined above, with a reducing agent capable of selectively reducing the 2-oxo group, and if desired subjecting the resulting compound of the formula I to one or more of the following optional finishing steps in any appropriate order:

a) reacting a compound of the formula I wherein $R^5$ is hydrogen with a diazoalkane having up to 6 (preferably up to 6) carbon atoms to yield a compound of the formula I wherein $R^5$ is an alkyl group having up to 6 carbon atoms;

b) reacting a compound of the formula I wherein $R^3$ and $R^5$ are hydrogen with an activated electrophile $R^3Q$ where $R^3$ is as defined herein and Q is an effective leaving group, especially a halogen atom, in particular chlorine or bromine;

c) isolating the final product of the formula I as such or as a pharmaceutically acceptable salt thereof as defined above.

The reducing agent may for example be lithium borohydride in an inert ether solvent, especially dioxan, or sodium bis(2-methoxyethoxy)-aluminum hydride (SMDA) in an inert organic solvent comprising an ether, especially dimethoxyethane-toluene, or tetrabutylammonium borohydride in an inert ether solvent, especially dioxan. The reduction of a compound of the formula IV with lithium borohydride is normally slow, and the reaction time can often be significantly reduced by silylation of the starting material of the formula IV. A preferred compound for use in the silylation step is 1,1,1,3,3,3,-hexamethyldisilazane (HMDS).

When SMDA is used as reducing agent, the reduction normally proceeds rapidly and in good yield, even when the starting material of the formula IV has not been silylated. However, SMDA should not be used for reducing compounds of the formula IV having fluorinated aryl substituents, especially a *p*-fluorophenyl group.

The reduction with tetrabutylammonium borohydride is efficacious but often slow.

Other reducing agents either are mostly ineffective or produce such a variety of products that separation of the resulting mixtures is unpractical. The above-mentioned reducing agents are the ones we have found effective. In view of the narrow range of reducing agents available for carrying out this selective reduction and the fact that reduction at the 2-position of the compounds of the formula IV (or their silylated derivatives) can indeed be effected selectively, this process ranks as an inventive process.

In step b), the activated electrophile of the formula $R^3Q$ may for example be a 3-halo-alkene, a 3-halo-alkyne, an α-halo ester, a benzyl halide, or an α-halo-acetonitrile. Substitutions (alkylations) using these electrophiles may be accomplished with a strong base and an inert (anhydrous) organic solvent, for example sodium hydride in N,N-dimethylformamide, triethylamine in acetone, or sodium or sodium ethoxide in ethanol. The compound of the formula $R^3Q$ can also be a tetraalkyl ammonium salt (for example the hydrogen sulphate) in a mixture of an inert organic solvent (such as methylene chloride) and an aqueous base (such as an alkali metal hydroxide, e.g. sodium hydroxide) under phase-transfer conditions; the tetraalkyl ammonium salt is preferably used in stoichiometric proportions. In this reaction the amine moiety $R^3_3N$ of the tetraalkyl ammonium salt functions as the leaving group Q.

Step b) is often a desirable final step in the preparation of compounds of formula I and stands in its own right as a feature of the invention.

The intermediates of the invention having structural formula IV wherein $R^5 = H$ may be prepared by reacting a correspondingly substituted compound having structural formula III with a dialkyl ($R^3$-malonate) in the presence of a stoichiometric amount of a base such as sodium hydride or sodium methoxide at an elevated temperature.

Compounds having structural formula III are prepared by reacting compounds having structural formula II with excess primary amine $R^4NH_2$ at elevated temperatures.

The intermediates of formula III may be prepared from readily-available starting materials according to the sequence of steps described below:

A)

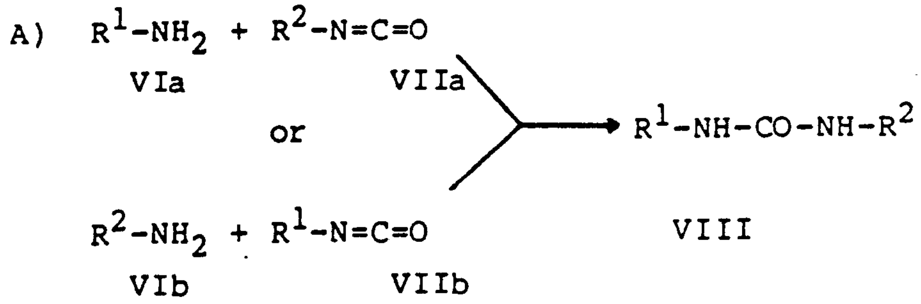


The ureas of formula VIII may be prepared by reacting approximately equimolar quantities of an amine ($R^1$—$NH_2$ or $R^2$—$NH_2$) with an isocyanate ($R^2$—N=C=O or $R^1$—N=C=O) in an inert solvent, e.g., chloroform.

B)

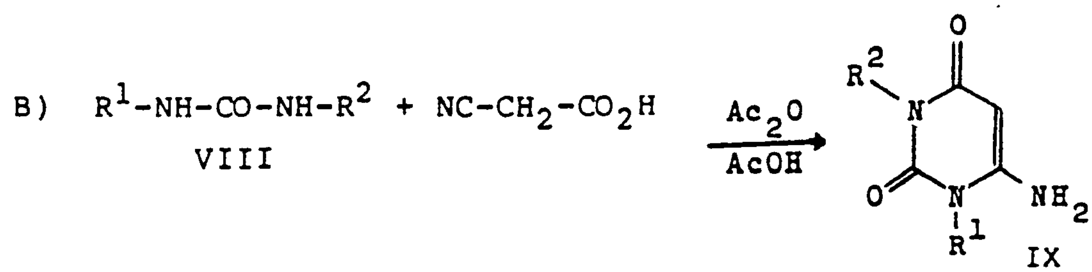


IX

Compounds of formula IX may be prepared by the well-known Traube purine synthesis or a modification thereof. Equimolar quantities of the compound of formula VIII and cyanoacetic acid are heated to 60°C with two equivalents of acetic anhydride using glacial acetic acid as solvent. After 2 to 8 hours as much as possible of the acetic acid and acetic anhydride are removed at 60°C in vacuo. The resultant mixture is poured into water and made basic, e.g., with solid sodium carbonate. The mixture is boiled 1—4 hours, then cooled. On standing either a solid will form which may be filtered off and purified, or an oil will form which may be extracted and purified.

Note that, for compounds of formula VIII where $R^1$ and $R^2$ are different, two different compounds of formula IX may be formed, i.e.,

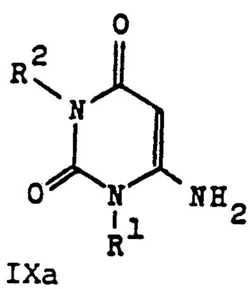

IXa

and

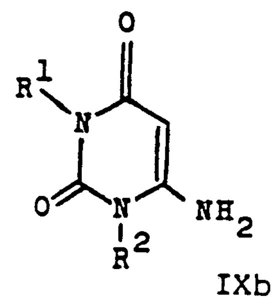

IXb

These compounds may be separated by fractional crystallization or by chromatography (e.g. column or HPLC).

C)

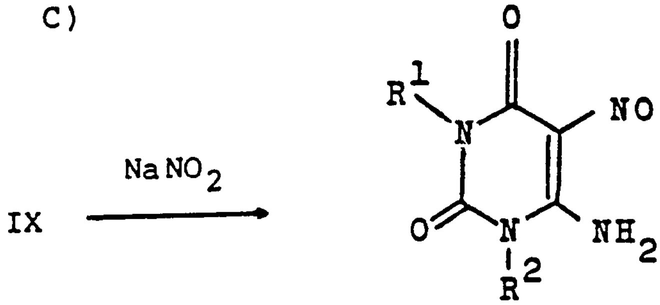


X

The purified 6-amino-uracil compounds of formula IX may be converted to the 5-nitroso-6-amino-uracil compounds of formula X by combining the 6-amino-uracil derivative and sodium nitrite (one equivalent) and boiling in ethanol/water while adding glacial acetic acid. The nitroso compound of formula X which precipitates is then filtered off, washed with water and dried.

D)

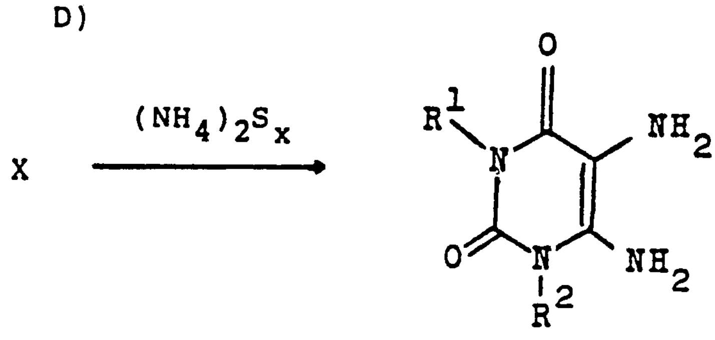


XI

The 6-amino-5-nitroso-uracil of formula X is reduced to the corresponding 5-amino-compound of formula XI in aqueous suspension by the use of an excess of ammonium polysulfide with warming. When the color is discharged, the mixture is cooled and the supernatant liquid is decanted off. The residue is dissolved in methylene chloride, which is dried and evaporated. The crude product is used in the next step.

E)

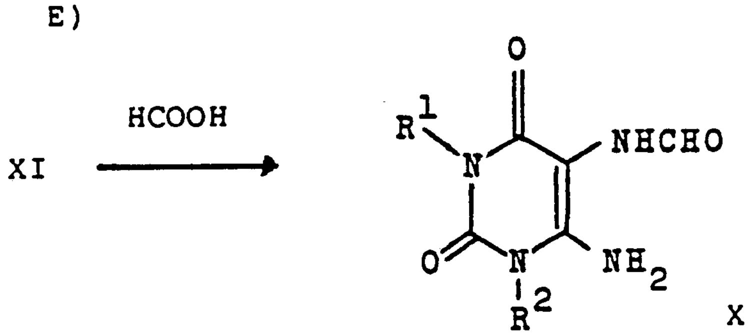


XII

The 5,6-diamino-uracil of formula XI is heated with excess formic acid at 120—150°C for 1—4 hours, then allowed to stand at room temperature overnight. Most of the acid is then removed (75°C; reduced pressure) and the residue is dissolved in hot methanol and filtered. The product of formula XII is isolated by chilling and filtering off the resulting solid or by evaporation of the methanol.

F)

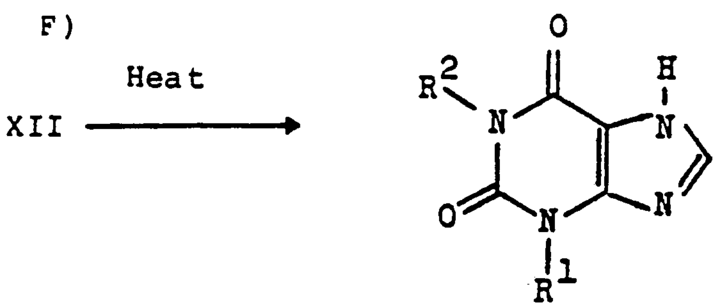


XIII

The 6-amino-5-formamido-uracil of formula XII is heated to 250—285°C until frothing ceases (10—60 mins.). The product is then cooled and the crude product of formula XIII is recrystallized, e.g., from MeOH/$H_2O$.

G)

XIII 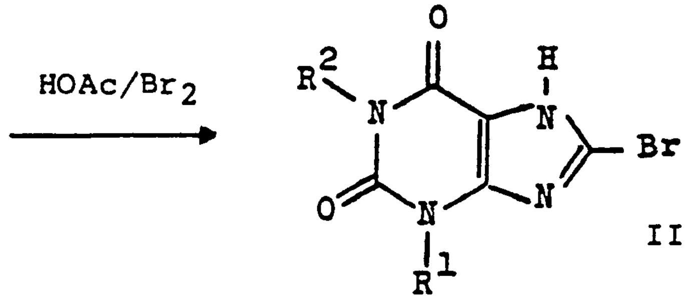


II

The xanthine compound of the formula XIII is dissolved in glacial acetic acid. The solution is warmed gradually to 100°C while a solution of bromine in acetic acid is slowly added until thin layer chromatography shows that the starting material has been consumed. The product, a compound of formula II, is isolated by pouring the reaction mixture into water, filtering and recrystallizing, if necessary.

The 8-bromoxanthine of formula II is converted into the 8-substituted-amino-xanthine of formula III by heating with excess amine at elevated temperatures as described in preparative Example 1, below. An 8-chloroxanthine can be used in this reaction instead of the 8-bromoxanthine, if desired.

The following Preparative Examples illustrate the preparation of the starting materials.

Preparative Example 1

8-Benzylamino-1,3-di-n-butyl-xanthine

Heat together a mixture of one equivalent of 8-bromo-1,3-di-n-butyl-xanthine and three to four equivalents of benzylamine at 160—180°C until thin layer chromatography shows that no starting xanthine remains. Cool. Triturate with ethanol and water to yield 8-benzylamino-1,3-di-n-butyl-xanthine.

Similarly, prepare other 8-(substituted amino)-1,3-disubstituted xanthines required for the preparation of the compounds of the present invention from the corresponding 8-bromo- (or 8-chloro)-1,3-disubstituted xanthines by heating with excess amine at elevated temperatures, in a sealed vessel if necessary.

Preparative Example 2A

9-Benzyl-1,3-dimethyl-7-(2-ethoxyethyl)-6-hydroxy-pyrimido[2,1-f]purine-2,4,8(1*H*,3*H*,9*H*)-trione (or tautomer)

To a stirred suspension of 7.43 g of 8-benzyl-aminotheophylline in 104 ml of dry N,N-dimethylformamide add portionwise over 10 minutes 1.19 g of a 60% dispersion of sodium hydride. Heat the mixture to 50°C under a nitrogen atmosphere for 30 minutes. Add 13.30 g of the diethyl ester of β-ethoxyethylmalonic acid. Heat the mixture to 150°C under a nitrogen atmosphere for approximately 37 hours. Allow the system to cool to room temperature and remove the solvent in vacuo. Add a mixture of water:chloroform (1:2.5) to the resulting semisolid. Acidify the aqueous portion with 3M HCl. Extract the product from the aqueous portion with chloroform. Wash the chloroform extracts with brine, dry over anhydrous sodium sulfate, filter and remove the solvent *in vacuo* to give the crude product. Triturate the crude product with ether. Purify the crude product by column chromatography on silica gel and triturate the major fraction with hexane to give the title compound, m.p. 156.5—157.5°C.

Preparative Example 2B

9-benzyl-1,3-dimethyl-6-hydroxy-7-(n-propyl)-pyrimido[2,1-f]purine-2,4,8(1*H*,3*H*,9*H*)-trione (or tautomer)

Suspend 8-benzylamino-theophylline (10 g) in diethyl n-propyl-malonate (65 ml). Add sodium methoxide (0.7 g), and stir and heat to about 200°C (bath temperature). Separate the ethanol which is formed with a Dean and Stark trap. After about 4 to 6 hours, raise the bath temperature to about 215°C until no more starting material is present (as shown by thin layer chromatography).

Cool to below 60°C and add ethanol. Stir and triturate and then filter, wash and dry in air. Recrystallize the product from acetonitrile (about 60 parts). Wash with ether and dry *in vacuo* at 70° to 75°C to yield the title compound having a melting point of 217°C (yield about 62%).

Preparative Example 3

1,3-Dimethyl-9-benzyl-6-methoxy-7-(n-propyl)-pyrimido[2,1-f]purine-2,4,8(1*H*,3*H*,9*H*)-trione

Dissolve 9-benzyl-1,3-dimethyl-6-hydroxy-7-(n-propyl)-pyrimido[2,1-f]purine-2,4,8(1*H*,3*H*,9*H*)-trione (3 g) in 200 ml chloroform at 0° and treat with an ethereal solution of diazomethane. Stir the solution at 0° for 1.5 hours and destroy the excess diazomethane by the addition of acetic acid. Wash the chloroform solution with a solution of sodium bicarbonate and remove the chloroform under reduced pressure. Chromatograph the solid obtained on silica gel using 1% methanol in chloroform to give the title compound, m.p. 199—201°C.

Preparative Example 4

9-Benzyl-1,3-dimethyl-6-hydroxy-pyrimido[2,1-f]purine-2,4,8(1*H*,3*H*,9*H*)-trione

Add 8-benzylaminotheophylline (30 g) and ethyl malonyl chloride (35.1 gm) to 600 ml of 1:1 dioxan/acetonitrile. Heat the reaction mixture to reflux under a nitrogen atmosphere until the 8-benzylaminotheophylline is consumed (ca. 3.5 hrs.). Cool the reaction mixture to room temperature and pour the solution into 800 ml of ether. Filter off the precipitate. Wash the precipitate with ether and dry the product to obtain the title compound, m.p. 205.5—209°C.

Similarly, prepare 1,3-Dimethyl-9-(4-fluorobenzyl)-6-hydroxy-pyrimido[2,1-f]purine-2,4,8(1*H*,3*H*,9*H*)-trione.

The following Examples illustrate the preparation of compounds of the invention. The denatured alcohol 2B used in some Examples is anhydrous ethanol denatured with 0.5% (v/v) benzene.

Example 1

9-benzyl-2,3-dihydro-1,3-dimethyl-6-hydroxy-7-propyl-pyrimido[2,1-f]purine-4,8(1*H*,9*H*)-dione

A. *Silylation.* Reflux a mixture of 30.1 g (0.076 mole) of 9-benzyl-1,3-dimethyl-6-hydroxy-7-propyl--pyrimido[2,1-f]purine-2,4,8(1*H*,3*H*,9*H*)-trione, 1.0 g of ammonium sulfate and 350 ml of 1,1,1,3,3,3-hexamethyldisilazane until the starting material dissolves, giving a cloudy solution. Distill the solvent under reduced pressure, and utilize the pinkish residual solid thus obtained directly in the reduction step "B".

B. *Reduction.* Dissolve the silylated product (approximately 0.076 mole) of step "A" in 1.3 liters of dry 1,4-dioxan. Place the reaction flask in a water bath at 15—20°C, and cautiously add 9.59 g (0.442 mole) of lithium borohydride portionwise to control the resultant frothing. Heat the reaction mixture carefully (because of foaming) to 90—95°C and maintain that temperature with effective stirring for 78 hours (disappearance of starting material may be monitored by TLC on silica with chloroform(80)-methanol(20)-concentrated ammonium hydroxide(1)).

Remove approximately one liter of dioxan by distillation under reduced pressure. Cool the residue to room temperature, and add 1 liter of chloroform. To the stirred mixture, cautiously add portionwise 200 ml of water, followed by 180 ml of 3M hydrochloric acid, and continue stirring for 0.5 hour. Separate the layers and extract the aqueous phase with two 200 ml portions of chloroform. Dry the combined extracts over sodium sulfate, remove the drying agent by filtration and remove the solvent from the filtrate at reduced pressure. Chromatograph the solid thus obtained on silica gel, eluting first with ethyl acetate(75)-hexanes(25), then with ethyl acetate, to obtain the title compound with m.p. 173—175°C. Recrystallize the chromatographed material to obtain product with m.p. 177—178.5°C.

*Sodium salt.* To a stirred suspension of 12.66 g (0.033 mole) of the chromatographed title compound in 1100 ml of water, add a solution of 1.33 g (0.033 mole) of sodium hydroxide in 400 ml of water. Stir for 5 hours; then filter the hazy, fine suspension through medium sintered glass. Lyophilize the clear filtrate to obtain the title salt as a solid. If the solid thus obtained is gummy, dissolve it in methanol; then remove methanol under reduced pressure, and triturate the residual solid with ether(1)-hexanes(3). Filter, and dry the product at 40°C under vacuum to obtain the sodium salt of the title compound as a 3/4 hydrate with m.p. 215°C (dec.).

C. Alternatively, preparation of the title compound may be carried out as follows:

To a stirred suspension of 0.5 g (1.27 mmoles) of 9-benzyl-2,3-dihydro-1,3-dimethyl-6-hydroxy-7-propyl--pyrimido[2,1-f]purine-2,4,8(1*H*,3*H*,9*H*)-trione in a mixture of 32 ml of dry dimethoxyethane and 12 ml of dry toluene, cautiously add 1.5 ml (5.1 mmoles) of a 3.4M solution of sodium bis(2-methoxyethoxy)aluminum hydride in toluene. Reflux the resultant mixture under a nitrogen atmosphere for 16 hours. Remove solvent under reduced pressure, and stir the residual oil under nitrogen with 20 ml of ether and 25 ml of 1.5M hydrochloric acid. Separate the layers, and extract the aqueous phase with two 20 ml volumes of ether. Dry the combined extracts over magnesium sulfate, filter off the drying agent and remove solvent from the filtrate under reduced pressure. Purify the residual solid chromatographically, as described above, to obtain the title compound.

Example 2

9-benzyl-2,3-dihydro-1,3-dimethyl-6-hydroxy-7-(3-methyl-2-butenyl)-pyrimido[2,1-f]purine-4,8(1*H*,9*H*)-dione

Step A: *9-Benzyl-2,3-dihydro-1,3-dimethyl-6-hydroxy-pyrimido[2,1-f]purine-4,8(*1H,9H*)-dione (I).*

To a suspension of 395 g (1.12 moles) of 9-benzyl-1,3-dimethyl-6-hydroxy-pyrimido[2,1-f]purine-2,4,8(1*H*,3*H*,9*H*)-trione in 10.5 liters of dry 1,4-dioxan, add 68.1 g (3.14 moles) of lithium borohydride in portions. Maintain the reaction temperature at 20—25°C by controlling the rate of addition and by use of a cooling bath as needed. Stir the mixture at room temperature for 0.5 hour, then reflux for 18 hours. Remove solvent under reduced pressure. Allow the residue to cool; then add 4.5 liters of chloroform. To the resultant mixture cautiously add dropwise 1.1 liters of water. Stir the mixture at room temperature until two clear phases result. Add 3N hydrochloric acid portionwise to bring the pH to 4—5. Separate the layers, and extract the aqueous phase with two 1.1 liter portions of chloroform. Wash the combined extracts with three 1.1 liter volumes of water, and dry over anhydrous sodium sulfate. Filter off the drying agent, and remove volatiles from the filtrate under reduced pressure. Recrystallize the residue from methanol-ethyl acetate to obtain title compound (*I*) as a solid with m.p. 176—182°C.

*Step B: Alkylation of I.* To a suspension of 75 g (0.221 mole) of *I* in 4.2 liters of ethanol (anhydrous; 2B) add 12 g (0.221 mole) of sodium methoxide portionwise during about twenty minutes. To the resultant mixture add 33 g (0.221) mole of 1-bromo-3-methyl-2-butene dropwise during 0.5 hour. Stir the reaction mixture for 18 hours at room temperature; then remove volatiles under reduced pressure. Pour the residue into 8.8 liters of cold water, saturate the aqueous phase with sodium chloride and extract with three 3 liter volumes of ether. Dry the combined extracts over anhydrous sodium sulfate, filter off the drying agent and remove solvent from the filtrate under reduced pressure. Chromatograph the residue on silica gel, eluting with ethyl acetate(3)-hexanes(2), to obtain the title compound as a solid with m.p. 153—154.5°C.

Alternatively, the alkylation of *I* may be carried out in the following manner: To a slurry of 0.85 g (0.0212 mole) of 60% sodium hydride (prewashed with hexanes) in 5 ml of dry N,N-dimethylformamide, add in two portions a solution of 6.11 g (0.018 mole) of *I* in 125 ml of dry N,N-DMF. Stir the mixture at room temperature under a nitrogen atmosphere for 15 minutes to obtain a clear solution. Add in one portion 4.06 g (0.0273 mole) of 1-bromo-3-methyl-2-butene (mild exotherm). Stir the reaction mixture under a nitrogen atmosphere at room temperature for 4.5 hours. Pour the reaction mixture into an ice-water mixture, and extract with four 150 ml portions of chloroform. Wash the combined extracts with water, dry them over anhydrous sodium sulfate, filter, evaporate off the solvent, and chromatograph the residue on silica gel, as described above, to obtain the title compound.

Example 3

2,3-Dihydro-1,3-dimethyl-9-(4-fluorobenzyl)-6-hydroxy-pyrimido[2,1-f]purine-4,8(1*H*,9*H*)-dione

Reflux a suspension of 395 g (1.06 moles) of 1,3-dimethyl-9-(4-fluorobenzyl)-6-hydroxy-pyrimido[2,1-f]purine-2,4,8(1*H*,3*H*,9*H*)-trione, 12.32 g of ammonium sulfate and 350 ml of 1,1,1,3,3,3-hexamethyldisilazane in 4 liters of chloroform until a clear solution is obtained (18—24 hr.). Remove chloroform and excess hexamethyldisilazane under reduced pressure, and treat the residual thick gum with 9.6 liters of dry 1,4-dioxan. While stirring the resultant mixture, cautiously add 70.4 g (3.24 moles) of lithium borohydride in portions under a stream of dry nitrogen. When foaming subsides, heat the mixture to 100°C for 18 hr. or until all starting material has been consumed (as determined by TLC on silica with chloroform(90)-methanol(10)-acetic acid(1)). Remove dioxan under reduced pressure, and stir the residue with 3 liters of chloform. Add 1.3 liters of water cautiously (because of foaming), followed by 2.3 liters of 3N hydrochloric acid. Stir for one hour; then separate the layers. Extract the aqueous phase with two 1.3 liter volumes of chloroform, and dry the combined extracts over anhydrous sodium sulfate. Filter off the drying agent, and remove solvent from the filtrate under reduced pressure. Dissolve the residual tacky solid in 1.5 liters of boiling acetonitrile, add a small amount of decolorizing carbon, reflux for 15 minutes, and filter through a pad of Celite. Chill the filtrate, and collect the resultant crystals. Wash the crystals with cold acetonitrile, and dry them under vacuum at 50°C to obtain the title compound with m.p. 214—232°C.

When the above reduction was carried out without pretreatment of the substrate with 1,1,1,3,3,3, hexamethyldisilazane, no reaction was observed after 6 days of reflux.

Example 4

2,3-dihydro-1,3-dimethyl-9-(4-fluorobenzyl)-6-hydroxy-7-(3-methyl-2-butenyl)-pyrimido[2,1-f]purine-4,8(1*H*,9*H*)-dione

Dissolve 1.48 g (0.0644 mole) of sodium metal in 450 ml of ethanol (2B; anhydrous). Add 23.0 g (0.0644 mole) of 2,3-dihydro-1,3-dimethyl-9-(4-fluorobenzyl)-6-hydroxy-pyrimido[2,1-f]purine-4,8(1*H*,9*H*)-dione. Stir the resultant suspension under a nitrogen atmosphere for 0.5 hour, then add 9.60 g (0.0644 mole) of 1-bromo-3-methyl-2-butene. Stir the mixture at room temperature for 90 hours under a nitrogen atmosphere. Filter off the white solids, and remove solvent from the filtrate under reduced pressure. Dissolve the residue in 150 ml of chloroform, add 125 ml of 3N hydrochloric acid and shake the mixture. Separate the layers, and extract the aqueous phase with two 50 ml volumes of chloroform. Dry the combined extracts over anhydrous magnesium sulfate, filter off the drying agent, and remove solvent from the filtrate under reduced pressure. Chromatograph the residual glassy solid on silica gel, eluting with ethyl acetate(3)-hexanes(1). Triturate the product thus obtained with hexane (125 ml per gram) and filter to obtain the title compound as a solid with m.p. 188—188.5°C.

Example 5

7,9-dibenzyl-2,3-dihydro-1,3-dimethyl-6-hydroxypyrimido[2,1-f]purine-4,8(1*H*,9*H*)-dione

To a suspension of 7.1 g (0.021 mole) of 9-benzyl-2,3-dihydro-1,3-dimethyl-6-hydroxy-pyrimido[2,1-f]purine-4,8(1*H*,9*H*)-dione in 200 ml of acetone, add 2.3 g (0.023 mole) of triethylamine, and stir the mixture for 5 minutes at room temperature under a nitrogen atmosphere to obtain a clear solution. Add dropwise to the solution 4.7 g (0.027 mole) of benzyl bromide, and reflux the mixture for 5 hours under a nitrogen atmosphere. Remove the acetone under reduced pressure, and triturate the gummy residue with methanol. Filter off the resultant white solid, pour the filtrate into water, acidify to pH 4—5 with dilute hydrochloric acid, and decant the aqueous supernatant. Dissolve the gummy residue in the chloroform, wash the solution with water, and dry over anhydrous magnesium sulfate. Remove the drying agent by filtration, and evaporate solvent from the filtrate under reduced pressure. Chromatograph the residual oil on silica gel, eluting with chloroform(96)-methanol(4), to obtain the title compound as a solid with m.p. 176—179°C.

*Sodium salt.* To a suspension of 0.5 g (0.012 mole) of 60% sodium hydride (prewashed with three 100 ml volumes of petroleum ether) in 300 ml of dry dimethoxyethane, add 3.4 g (0.0079 mole) of analytically pure 7,9-dibenzyl-2,3-dihydro-1,3-dimethyl-6-hydroxy-pyrimido[2,1-f]purine-4,8(1*H*,9*H*)-dione. Stir the mixture for 30 minutes at room temperature under a nitrogen atmosphere. Filter off excess sodium hydride. Concentrate the filtrate under reduced pressure to an oil and add ether to precipitate a solid. Isolate the solid by filtration, and triturate it in fresh ether. Filter again, and dry the solid at 70°C under reduced pressure to obtain the hemihydrate salt of the title compound as a yellow powder with m.p. 175—185°C.

The compounds of this invention can be used to treat inflammatory conditions such as arthritis, spondylitis and tendonitis and are conventionally formulated for oral, parenteral, topical and transdermal use.

The anti-inflammatory potential of the compounds of the present invention may be determined by Prophylactic Adjuvant-Induced Arthritis in Rats (AAR), as set forth below.

Of course, the dosage regimen and amount to be administered and mode of administration depend upon the judgement of the attending clinician considering the potency of the particular compound used, the age and general health of the patient and the severity of the inflammatory condition. Generally the recommended regimen is a dosage range of about 1 milligram per kilogram of body weight per day to about 50 miligrams per kilogram of body weight per day in divided doses taken at about 4 hour intervals.

Prophylactic Adjuvant-Induced Arthritis in Rats (AAR)

Groups of 10 male Lewis rats (from Charles River Laboratories, Ma.), weighing 150—170 grams are sensitized by subplantar injection in the left hind paw with 0.1 ml Freund's complete adjuvant enriched with heat-killed tuberculin bacilli. Hind paw volumes are determined with a mercury plethysmograph from Day 0 to Day 21 of the study. Differences in paw volume on Day 0 and Day 21 are recorded as the delta ($\Delta$) paw volume. In sensitized rats the injected hind paw increases in size by Day 2 and seven days later a similar response is seen in the contralateral hind paw. Differences in body weights on Day 0 and Day 21 are recorded as the delta ($\Delta$) body weight gain.

Daily oral doses of the drug suspended in methylcellulose or of methylcellulose alone are administered from Day 0 to Day 21.

An exemplifying compound of the invention, 9-benzyl-2,3-dihydro-1,3-dimethyl-6-hydroxy-7-(3-methyl-2-butenyl)-pyrimido[2,1-f]purine-4,8(1*H*,9*H*)-dione, was found to have an $ED_{50}$ in the rat AAR test of 7.5 mg/kg and to show little toxicity at doses up to 300 mg/kg.

The compounds of this invention are also useful for the treatment of allergy-caused diseases and their preferred use is for treating allergic chronic obstructive lung diseases. Chronic obstructive lung disease as used herein means disease conditions in which the passage of air through the lungs is obstructed or diminished such as is the case in asthma, bronchitis.

The compounds of this invention can be administered in unit dosage forms such as tablets, capsules or pills, or in powders, granules, sterile parenteral solutions or suspensions, mechanical delivery devices, e.g., transdermals. In whatever form the compounds are dispensed, they may be admixed with pharmaceutically acceptable excipients, binders, dispersing agents and carriers generally used in the art.

Exemplary of the pharmaceutical carriers, excipients, preservatives and binders are gelatin, lactose, starch, magnesium stearate, talc, vegetable oils, gums, polyalkylene glycols, etc. The pharmaceutical dosage forms are prepared by the methods conventionally used in the art. Further, the dosage units may also contain a compatible anti-depressant and/or analgesic to treat the depression and pain usually associated with chronic inflammatory conditions.

Topical pharmaceutical forms may be prepared according to procedures well known in the art, and may contain a variety of ingredients as carriers. Formulations for topical use include ointments, creams, gels, lotions, powders, aerosols, sprays, and transdermal devices such as adhesive pads. Ointments, lotions and creams may contain water, oils, fats, waxes, polyesters, alcohols, or polyols, and fragrances, emulsifiers and preservatives. Powders are made by mixing the active ingredient with a readily available, inert, pulverous distributing agent, such as talcum, calcium carbonate, tricalcium phosphate, or boric acid. Aqueous suspensions of these powders may also be made. Solutions or emulsions may be prepared using inert solvents which are preferably nonflammable, odorless, colorless, and non-toxic, for example vegetable oils, isopropanol, dimethyl sulfoxide, hydrogenated naphthalenes and alkylated naphthalenes. Similarly, aerosol or non-aerosol sprays may be prepared using solutions or suspensions in appropriate solvents, e.g., difluorodichloromethane for aerosols.

Topical formulations, e.g., ointments, creams, lotions, powders, or sprays, will usually contain about 0.1 to 3 grams of compound of formula I per 100 grams of carrier.

The following examples illustrate the preparation of solid dosage forms; the active ingredient (compound of formula I) is preferably 9-benzyl-2,3-dihydro-1,3-dimethyl-6-hydroxy-7-(3-methyl-2-butenyl)-pyrimido[2,1-f]purine-4,8(1*H*,9*H*)-dione or its sodium salt, but an equivalent amount of another compound of formula I can if desired be substituted:

A. Capsules:

| No. | Ingredient | mg/capsule | mg/capsule |
|---|---|---|---|
| 1 | Compound of the invention | 50 | 250 |
| 2 | Lactose USP | 50 | 100 |
| 3 | Corn Starch, Food Grade | 48.5 | 50 |
| 4 | Microcrystalline Cellulose NF | 50 | 95 |
| 5 | Magnesium Stearate NF | 1.5 | 5 |
| | Total | 200 | 500 |

Method of Manufacture

Mix Item Nos. 1, 2, 3 and 4 in a suitable mixer for 10—15 minutes. Add Item No. 5 and mix for 1—3 minutes. Fill the mixture into suitable two-piece hard gelatin capsules using an encapsulating machine.

B. Tablets:

| No. | Ingredient | mg/tablet | mg/tablet |
|---|---|---|---|
| 1 | Compound of the invention | 50 | 250 |
| 2 | Lactose USP | 68 | 57 |
| 3 | Corn Starch, Food Grade, as a 10% paste in Purified Water | 10 | 20 |
| 4 | Corn Starch, Food Grade | 20 | 18 |
| 5 | Magnesium Stearate NF | 2 | 5 |
| | Total | 150 | 350 |

Method of Manufacture

Mix Item Nos. 1 and 2 in a suitable mixer for 10—15 minutes. Granulate the mixture with Item No. 3. Pass if needed, dry the wet granules and then mill them. Combine Item No. 4 and the dried granules and mix for 10—15 minutes. Add Item No. 5 and mix for 1—3 minutes. Compress the mixture to appropriate size and weight on a suitable tablet machine.

## Claims

1. A compound having the structural formula I

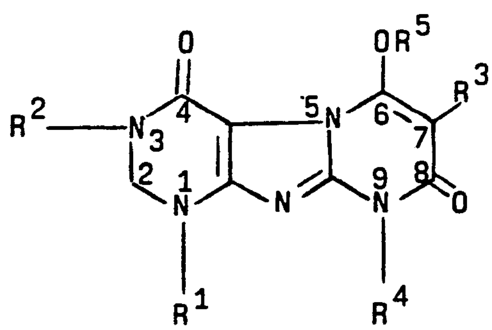

I

including the tautomers and pharmaceutically acceptable salts, wherein

$R^1$ and $R^2$ are independently selected from hydrogen, cycloalkyl having from 3 to 8 carbon atoms, phenyl, substituted phenyl, lower alkyl, and lower alkyl substituted with cycloalkyl having from 3 to 8 carbon atoms, with phenyl, with thienyl or with substituted phenyl;

$R^3$ is selected from hydrogen, formyl, cycloalkyl having from 3 to 8 carbon atoms, alkenyl having from 2 to 8 carbon atoms, alkenyl having from 3 to 8 carbon atoms and substituted with up to 6 fluorines, alkynyl having from 3 to 8 carbon atoms, cycloalkenyl having from 5 to 8 carbon atoms, acyloxyalkyl having from 2 to 12 carbon atoms, phenyl, substituted phenyl, lower alkyl, X—$R^6$, -alkyl—Y—$C_pH_{2p+1}$ wherein the alkyl portion has 1 to 6 carbon atoms, —$(CH_2)_nCONR^7R^8$, —$(CH_2)_mCO$—$OR^9$, and lower alkyl substituted with hydroxy, with sulfhydryl, with cyano, with amino, with halo, with cycloalkyl having from 3 to 8 carbon atoms, with phenyl, with thienyl or with substituted phenyl;

wherein X is 0, NH or S;

$R^6$ is phenyl, substituted phenyl, lower alkyl, or lower alkyl substituted with cycloalkyl having from 3 to 8 carbon atoms or with phenyl;

p is an integer from 0 to 4;

Y represents CO, O, S, $S^+$—$O^-$, $SO_2$ or $=NC_rH_{2r+1}$ wherein r is an integer from 0 to 4, with the proviso that p is an integer from 1 to 4 when Y is $S^+$—$O^-$ or $SO_2$;

$R^7$ and $R^8$ are independently hydrogen or lower alkyl;

n is an integer from 0 to 6;

$R^9$ is a hydrogen or lower alkyl;

and m is an integer from 0 to 6;

$R^4$ is selected from hydrogen, phenyl, thienyl, pyridyl, substituted phenyl, lower alkyl, and lower alkyl substituted with cycloalkyl having from 3 to 8 carbon atoms, with phenyl, with thienyl or with substituted phenyl; and

$R^5$ is selected from hydrogen and alkyl having from 1 to 4 carbon atoms;

the term "substituted" in relation to phenyl, pyridyl and thienyl groups means such groups substituted with 1 to 3 substituents independently selected from halogen, trifluoromethyl, $CONH_2$, —$CO_2H$, hydroxy, —$S(O)_aR^{10}$ wherein $R^{10}$ is lower alkyl and a is 0, 1 or 2, —$OR^{11}$ wherein $R^{11}$ is lower alkyl, and $COR^{12}$ wherein $R^{12}$ is lower alkyl or alkoxy having from 1 to 6 carbon atoms;

and the term "lower" in relation to alkyl groups indicates such groups having from 1 to 6 carbon atoms.

2. A compound as claimed in claim 1 having the structural formula I and the pharmaceutically acceptable salts thereof, wherein

$R^1$ and $R^2$ are independently selected from alkyl having from 1 to 4 carbon atoms;

$R^3$ is hydrogen, alkenyl, having from 2 to 8 carbon atoms which may be substituted with up to 6 fluorines, alkynyl having from 3 to 8 carbon atoms, cycloalkenyl having from 5 to 8 carbon atoms, lower alkyl, or lower alkyl substituted with phenyl;

$R^4$ is lower alkyl which is substituted with phenyl, with thienyl or with substituted phenyl; and

$R^5$ is hydrogen.

3. A compound as claimed in claim 2 wherein $R^1$ and $R^2$ are alkyl having 1 to 3 carbon atoms, preferably both methyl.

4. A compound as claimed in any of claims 1 to 3 wherein $R^3$ is hydrogen, alkenyl having from 3 to 8 carbon atoms which may be substituted with up to 6 fluorines, alkynyl having from 3 to 8 carbon atoms, cycloalkenyl having from 5 to 8 carbon atoms, lower alkyl or lower alkyl substituted with phenyl.

5. A compound as claimed in claim 4 wherein $R^3$ is hydrogen methyl, n-propyl, 2-propynyl, allyl, trans-2-butenyl, 2-cyclohexenyl, —$CH_2CH=C(CH_3)_2$, —$CH_2CH=C(CF_3)CH_3$, —$CH_2CH=C(CF_3)_2$ or benzyl.

6. A compound as claimed in any of claims 1 to 5 wherein $R^4$ is benzyl or substituted benzyl, where "substituted" has the meaning given in claim 1 in relation to phenyl, especially p-fluorobenzyl, or 2-thienylmethyl.

A. Compound as claimed in claim 1 selected from:

9-benzyl-2,3-dihydro-1,3-dimethyl-6-hydroxy-7-propyl-pyrimido[2,1-f]purine-4,8(1*H*,9*H*)-dione;

9-benzyl-2,3-dihydro-1,3-dimethyl-6-hydroxy-pyrimido[2,1-f]purine-4,8(1*H*,9*H*)-dione;

9-benzyl-2,3-dihydro-1,3-dimethyl-6-hydroxy-7-methyl-pyrimido[2,1-f]purine-4,8(1*H*,9*H*)-dione;

7,9-dibenzyl-2,3-dihydro-1,3-dimethyl-6-hydroxy-pyrimido[2,1-f]purine-4,8(1*H*,9*H*)-dione;

9-benzyl-2,3-dihydro-1,3-dimethyl-7-formyl-6-hydroxy-pyrimido[2,1-f]purine-4,8(1*H*,9*H*)-dione;

9-benzyl-2,3-dihydro-1,3-dimethyl-7-(2-propynyl)-6-hydroxy-pyrimido[2,1-f]purine-4,8(1*H*,9*H*)-dione;

9-benzyl-7-(trans-2-butenyl)-2,3-dihydro-1,3-dimethyl-6-hydroxy-pyrimido[2,1-f]purine-4,8(1*H*,9*H*)-dione;

9-benzyl-7-(3-cyclohexenyl)-2,3-dihydro-1,3-dimethyl-6-hydroxy-pyrimido[2,1-f]purine-4,8(1*H*,9*H*)-dione;

9-benzyl-2,3-dihydro-1,3-dimethyl-7-(ethoxycarbonylmethyl)-6-hydroxy-pyrimido[2,1-f]purine-4,8(1*H*,9*H*)-dione;

and especially

9-benzyl-2,3-dihydro-1,3-dimethyl-6-hydroxy-7-(3-methyl-2-butenyl)-pyrimido[2,1-f]purine-4,8(1*H*,9*H*)-dione;

and their sodium salts.

8. A compound as claimed in claim 1 selected from:

2,3-dihydro-1,3-dimethyl-9-(4-fluorobenzyl)-6-hydroxy-7-propyl-pyrimido[2,1-f]purine-4,8(1*H*,9*H*)-dione;

2,3-dihydro-1,3-dimethyl-9-(4-fluorobenzyl)-6-hydroxy-7-(3-methyl-2-butenyl)-pyrimido[2,1-f]purine-4,8(1*H*,9*H*)-dione;

2,3-dihydro-1,3-dimethyl-9-(4-fluorobenzyl)-6-hydroxy-pyrimido[2,1-f]purine-4,8(1*H*,9*H*)-dione;

2,3-dihydro-1,3-dimethyl-9-(4-fluorobenzyl)-6-hydroxy-7-(2-propenyl)-pyrimido[2,1-f]purine-4,8(1*H*,9*H*)-dione;

2,3-dihydro-1,3-dimethyl-9-(4-methoxybenzyl)-6-hydroxy-7-(3-methyl-2-butenyl)-pyrimido[2,1-f]purine-4,8(1*H*,9*H*)-dione;

and their sodium salts.

9. A compound claimed in claim 1, namely

2,3-dihydro-1,3-dimethyl-9-(2-thienylmethyl)-6-hydroxy-7-propyl-pyrimido[2,1-f]purine-4,8(1*H*,9*H*)-dione;

and its sodium salt.

10. A process for the preparation of compounds of the formula I defined in claim 1, which comprises reducing a compound of the formula IV

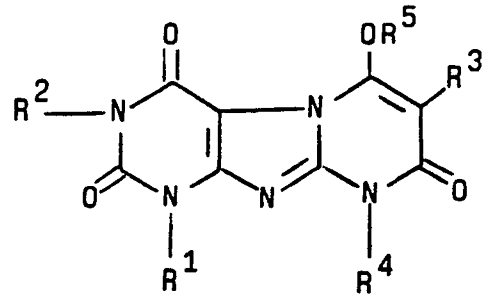

IV

or a silylated derivative thereof formed with 1,1,1,3,3,3-hexamethyldisilazane, wherein $R^1$, $R^2$, $R^3$, $R^4$ and $R^5$ are as defined in claim 1, with a reducing agent that is capable of selectively reducing the 2-oxo group;

and if desired subjecting the resulting compound of the formula I to one or more of the following optional finishing steps in any appropriate order:

a) reacting a compound of the formula I wherein $R^5$ is hydrogen with a diazoalkane having up to 6 carbon atoms to yield a compound of the formula I wherein $R^5$ is an alkyl group having up to 6 carbon atoms;

b) reacting a compound of the formula I wherein $R^3$ and $R^5$ are hydrogen with an activated electrophile $R^3Q$ where $R^3$ is as defined herein and Q is an effective leaving group, especially a halogen atom, in particular chlorine or bromine;

c) isolating the final product of the formula I as such or as a pharmaceutically acceptable salt thereof as defined above.

11. A process as claimed in claim 10, wherein the reducing agent is lithium borohydride in an inert ether solvent, or sodium bis(2-methoxyethoxy)-aluminum hydride in an inert organic solvent comprising an ether, or tetrabutylammonium borohydride in an inert ether solvent.

12. A process as claimed in claim 10 or claim 11 wherein, in step b), the activated electrophile of the formula $R^3Q$ is a 3-halo-alkene, a 3-halo-alkyne, an a-halo ester, a benzyl halide, or an a-halo-acetonitrile, in the presence of a strong base and inert organic solvent, or a tetraalkyl ammonium salt in a mixture of an inert organic solvent under phase-transfer conditions.

13. A process for preparing a compound of the formula I defined in claim 1 which comprises reacting a compound of the formula Ia

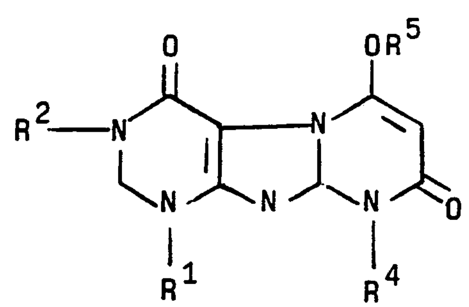

Ia

wherein $R^1$, $R^2$, $R^4$ and $R^5$ are as defined in claim 1, with an activated electrophile capable of introducing the $R^3$ group.

14. The process of claim 13 wherein the activated electrophile is a 3-halo-alkene, 3-halo-alkyne, a-halo ester, benzyl halide or a-halo-acetonitrile.

15. A pharmaceutical composition which comprises a compound of formula I defined in claim 1, or a tautomer or pharmaceutically acceptable salt thereof, in combination with a pharmaceutically acceptable carrier.

16. Use of a compound of formula I defined in claim 1, or a tautomer or pharmaceutically acceptable salt thereof, for the preparation of pharmaceutical compositions useful in the treatment of inflammation in a mammal.

**Patentansprüche**

1. Eine Verbindung der Strukturformel I

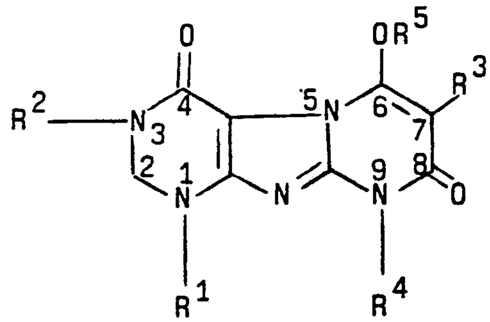

I

einschließlich der Tautomeren und der pharmazeutisch annehmbaren Salze derselben, worin

$R^1$ und $R^2$ unabhängig voneinander aus Wasserstoff, Cycloalkyl mit 3 bis 8 Kohlenstoff-Atomen, Phenyl, substituiertem Phenyl, Niederalkyl und Niederalkyl, das mit Cycloalkyl mit 3 bis 8 Kohlenstoff-Atomen, mit Phenyl, mit Thienyl oder mit substituiertem Phenyl substituiert ist, ausgewählt ist,

$R^3$ aus Wasserstoff, Formyl, Cycloalkyl mit 3 bis 8 Kohlenstoff-Atomen, Alkenyl mit 2 bis 8 Kohlenstoff-Atomen, Alkenyl mit 3 bis 8 Kohlenstoff-Atomen und substituiert mit bis zu 6 Fluor, Alkinyl mit 3 bis 8 Kohlenstoff-Atomen, Cycloalkenyl mit 5 bis 8 Kohlenstoff-Atomen, Acyloxyalkyl mit 2 bis 12 Kohlenstoff-Atomen, Phenyl, substituiertem Phenyl, Niederalkyl, X—$R^6$, -Alkyl—Y—$C_pH_{2p+1}$ worin der Alkyl-Teil 1 bis 6 Kohlenstoff-Atome hat, $—(CH_2)_nCONR^7R^8$, $—(CH_2)_mCO—OR^9$ und Niederalkyl, das mit Hydroxy, mit Sulfhydryl, mit Cyano, mit Amino, mit Halogen, mit Cycloalkyl mit 3 bis 8 Kohlenstoff-Atomen, mit Phenyl, mit Thienyl oder mit substituiertem Phenyl substituiert ist, ausgewählt ist,

worin

X O, NH oder S ist,

$R^6$ Phenyl, substituiertes Phenyl, Niederalkyl oder Niederalkyl, das mit Cycloalkyl mit 3 bis 8 Kohlenstoff-Atomen oder mit Phenyl substituiert ist, ist,

p eine ganze Zahl von 0 bis 4 ist,

Y CO, O, S, $S^+—O^-$, $SO_2$ oder $=NC_rH_{2r+1}$, worin r eine ganze Zahl von 0 bis 4 ist, ist, mit der Maßgabe, daß p eine ganze Zahl von 1 bis 4 ist, wenn Y $S^+—O^-$ oder $SO_2$ ist,

$R^7$ und $R^8$ unabhängig voneinander Wasserstoff oder Niederalkyl sind,

n eine ganze Zahl von 0 bis 6 ist,

$R^9$ Wasserstoff oder Niederalkyl ist und

m eine ganze Zahl von 0 bis 6 ist,

$R^4$ aus Wasserstoff, Phenyl, Thienyl, Pyridyl, substituiertem Phenyl, Niederalkyl und Niederalkyl, das mit Cycloalkyl mit 3 bis 8 Kohlenstoff-Atomen, mit Phenyl, mit Thienyl oder mit substituiertem Phenyl substituiert ist, ausgewählt ist und

$R^5$ aus Wasserstoff und Alkyl mit 1 bis 4 Kohlenstoff-Atomem ausgewählt ist,

wobei der Begriff "substituiert" in Verbindung mit Phenyl-, Pyridyl- und Thienyl-gruppen solche Gruppen bezeichnet, die mit 1 bis 3 Substituenten substituiert sind, die unabhängig voneinander aus Halogen, Trifluoromethyl, $CONH_2$, $—CO_2H$ Hydroxy, $—S(O)_aR^{10}$, worin $R^{10}$ eine Niederalkyl ist und a 0, 1 oder 2 ist, $—OR^{11}$, worin $R^{11}$ Niederalkyl ist, und $COR^{12}$, worin $R^{12}$ Niederalkyl oder Alkoxy mit 1 bis 6 Kohlenstoff-Atomen ist, und

der Begriff "Nieder" in Verbindung mit Alkyl-Gruppen solche Gruppen mit 1 bis 6 Kohlenstoff-Atomen bezeichnet.

2. Verbindung nach Anspruch 1 mit der Strukturformel I und deren pharmazeutisch annehmbare Salze, worin

$R^1$ und $R^2$ unabhängig voneinander aus Alkyl mit 1 bis 4 Kohlenstoff-Atomen ausgewählt sind,

$R^3$ Wasserstoff, Alkenyl mit 2 bis 8 Kohlenstoff-Atomen, das mit bis zu 6 Fluor substituiert sein kann, Alkinyl mit 3 bis 8 Kohlenstoff-Atomen, Cycloalkenyl mit 5 bis 8 Kohlenstoff-Atomen, Niederalkyl oder Niederalkyl, das mit Phenyl substituiert ist, ist,

$R^4$ Niederalkyl, das mit Phenyl, mit Thienyl oder mit substituiertem Phenyl substituiert ist, ist und $R^5$ Wasserstoff ist.

3. Verbindung nach Anspruch 2, worin $R^1$ und $R^2$ Alkyl mit 1 bis 3 Kohlenstoff-Atomen, vorzugsweise beide Methyl, sind.

4. Verbinding nach irgendeinem der Ansprüche 1 bis 3, worin $R^3$ Wasserstoff, Alkenyl mit 3 bis 8 Kohlenstoff-Atomen, das mit bis zu 6 Fluor substituiert sein kann, Alkinyl mit 3 bis 8 Kohlenstoff-Atomen, Cycloalkenyl mit 5 bis 8 Kohlenstoff-Atomen, Niederalkyl, oder Niederalkyl, das mit Phenyl substituiert ist, ist.

5. Verbindung nach Anspruch 4, worin $R^3$ Wasserstoff, Methyl, n-Propyl, 2-Propinyl, Allyl, trans-2-Butenyl, 2-Cyclohexenyl, $—CH_2CH=C(CH_3)_2$, $—CH_2CH=C(CF_3)CH_3$, $—CH_2CH=C(CF_3)_2$ oder Benzyl ist.

6. Verbindung nach irgendeinem der Ansprüche 1 bis 5, worin $R^4$ Benzyl oder substituiertes Benzyl ist, wobei der Begriff "substituiert" die in Anspruch 1 in Verbindung mit Phenyl angegebene Bedeutung hat, insbesondere p-Fluorobenzyl oder 2-Thienylmethyl.

7. Verbindung nach Anspruch 1, ausgewählt aus
9-Benzyl-2,3-dihydro-1,3-dimethyl-6-hydroxy-7-propyl-pyrimido[2,1-f]purin-4,8(1*H*,9*H*)-dion;
9-Benzyl-2,3-dihydro-1,3-dimethyl-6-hydroxy-pyrimido[2,1-f]purin-4,8(1*H*,9*H*)-dion;
9-Benzyl-2,3-dihydro-1,3-dimethyl-6-hydroxy-7-methyl-pyrimido[2,1-f]purin-4,8(1*H*,9*H*)-dion;
7,9-Dibenzyl-2,3-dihydro-1,3-dimethyl-6-hydroxy-pyrimido[2,1-f]purin-4,8(1*H*,9*H*)-dion;
9-Benzyl-2,3-dihydro-1,3-dimethyl-7-formyl-6-hydroxy-pyrimido[2,1-f]purin-4,8(1*H*,9*H*)-dion;
9-Benzyl-2,3-dihydro-1,3-dimethyl-7-(2-propinyl)-6-hydroxy-pyrimido[2,1-f]purin-4,8(1*H*,9*H*)-dion;
9-Benzyl-7-(trans-2-butenyl)-2,3-dihydro-1,3-dimethyl-6-hydroxy-pyrimido[2,1-f]purin-4,8(1*H*,9*H*)-dion;
9-Benzyl-7-(3-cyclohexenyl)-2,3-dihydro-1,3-dimethyl-6-hydroxy-pyrimido[2,1-f]purin-4,8(1*H*,9*H*)-dion;
9-Benzyl-2,3-dihydro-1,3-dimethyl-7-(ethoxycarbonylmethyl)-6-hydroxy-pyrimido[2,1-f]purin-4,8(1*H*,9*H*)-dion;
und insbesondere
9-Benzyl-2,3-dihydro-1,3-dimethyl-6-hydroxy-7-(3-methyl-2-butenyl)-pyrimido[2,1-f]purin-4,8(1*H*,9*H*)-dion;
und deren Natrium-Salze.

8. Verbindung nach Anspruch 1, ausgewählt aus
2,3-Dihydro-1,3-dimethyl-9-(4-fluorobenzyl)-6-hydroxy-7-propyl-pyrimido[2,1-f]purin-4,8(1*H*,9*H*)-dion;
2,3-Dihydro-1,3-dimethyl-9-(4-fluorobenzyl)-6-hydroxy-7-(3-methyl-2-butenyl)-pyrimido[2,1-f]purin-4,8(1*H*,9*H*)-dion;
2,3-Dihydro-1,3-dimethyl-9-(4-fluorobenzyl)-6-hydroxy-pyrimido[2,1-f]purin-4,8(1*H*,9*H*)-dion;
2,3-Dihydro-1,3-dimethyl-9-(4-fluorobenzyl)-6-hydroxy-7-(2-propenyl)-pyrimido[2,1-f]purin-4,8(1*H*,9*H*)-dion;
2,3-Dihydro-1,3-dimethyl-9-(4-methoxybenzyl)-6-hydroxy-7-(3-methyl-2-butenyl)-pyrimido[2,1-f]purin-4,8(1*H*,9*H*)-dion;
und deren Natrium-Salze.

9. Verbindung nach Anspruch 1, nämlich
2,3-Dihydro-1,3-dimethyl-9-(2-thienylmethyl)-6-hydroxy-7-propyl-pyrimido[2,1-f]purin-4,8(1*H*,9*H*)-dion;
und ihr Natrium-Salz.

10. Verfahren zur Herstellung von Verbindungen Formel I nach Anspruch 1, umfassend die Reduktion einer Verbindung der Formel IV

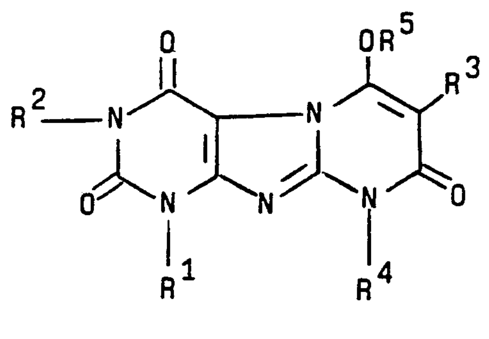

IV

oder eines silylierten Derivats derselben, das mit 1,1,1,3,3,3-Hexamethyldisilazan gebildet ist, worin $R^1$, $R^2$, $R^3$, $R^4$ und $R^5$ die in Anspruch 1 angegebenen Bedeungen haben, mit einem Reduktionsmittel, das befähigt ist, die 2-Oxo-Gruppe selektiv zu reduzieren; und

gewünschtenfalls das Einwirkenlassen eines oder mehrerer der folgenden wahlfreien Nachbearbeitungsschritte, in beliebiger sinnvoller Reihenfolge, auf die resultierende Verbindung der Formel I:

a) Umsetzung einer Verbindung der Formel I, in der $R^5$ Wasserstoff ist, mit einem Diazoalkan mit bis zu 6 Kohlenstoff-Atomen zur Bildung einer Verbindung der Formel I, in der $R^5$ eine Alkyl-Gruppe mit bis zu 6 Kohlenstoff-Atomen ist;

b) Umsetzung einer Verbindung der Formel I, in der $R^3$ und $R^5$ Wasserstoff sind, mit einem aktivierten Elektrophil $R^3Q$, worin $R^3$ die hierin angegebenen Bedeutungen hat und Q eine wirksame abspaltbare Gruppe, insbesondere ein Halogen-Atom, speziell Chlor oder Brom, ist,

c) Isolieren des Endproduktes der Formel I als solches oder als pharmazeutisch annehmbares Salz desselben, wie es im Vorstehenden definiert ist.

11. Verfahren nach Anspruch 10, worin das Reduktionsmittel Lithiumborhydrid in einem inerten Ether-Lösungsmittel oder Natrium-bis(2-methoxyethoxy)aluminiumhydride in einem einen Ether umfassenden inerten organischen Lösungsmittel oder Tetrabutylammoniumborhydrid in einem inerten Ether-Lösungsmittel ist.

12. Verfahren nach Anspruch 10 oder Anspruch 11, worin in Schritt b) das aktivierte Elektrophil $R^3Q$ ein 3-Halogenoalken, ein 3-Halogenoalkin, ein a-Halogenoester, ein Benzylhalogenid oder ein a-Halogenoacetonitril in Gegenwart einer starken Base und eines inerten Lösungsmittels oder ein Tetraalkylammonium-Salz in einem Gemisch eines inerten organischen Lösungsmittels unter Phasen-Transfer-Bedingungen ist.

13. Verfahren zur Herstellung einer Verbindung der Formel I nach Anspruch 1, umfassend die Reaktion einer Verbindung der Formel Ia

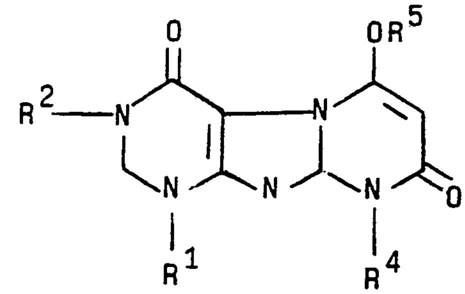

Ia

worin $R^1$, $R^2$, $R^4$ und $R^5$ die in Anspruch 1 angegebenen Bedeutungen haben, mit einem mit einem zur Einführung der $R^3$-Gruppe befähigten aktivierten Elektrophil.

14. Verfahren nach Anspruch 13, worin das aktivierte Elektrophil ein 3-Halogenoalken, ein 3-Halogenoalkin, ein a-Halogenoester, ein Benzylhalogenid oder ein a-Halogenoacetonitril ist.

15. Pharmazeutische Zusammensetzung, umfassend eine Verbindung der Formel I nach Anspruch 1 oder ein Tautomer oder pharmazeutisch annehmbares Salz derselben in Kombination mit einem pharmazeutisch annehmbaren Träger.

16. Verwendung einer Verbindung der Formel I nach Anspruch 1 oder eines Tautomers oder pharmazeutisch annehmbaren Salzes derselben zur Herstellung einer pharmazeutischen Zusammensetzung, die zur Behandlung von Entzündungen bei Säugern von Nutzen ist.

**Revendications**

1. Composé ayant la formule de structure I

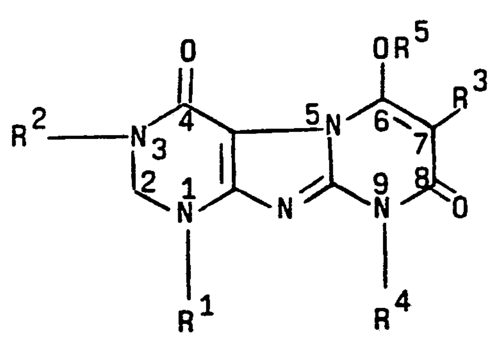

I

comprenant leurs tautomères et leurs sel acceptables en pharmacie, où

$R^1$ et $R^2$ sont indépendamment choisis parmi hydrogène, cycloalkyle ayant de 3 à 8 atomes de carbone, phényle, phényle substitué, alkyle inférieur et alkyle inférieur substitué par cycloalkyle ayant 3 à 8 atomes de carbone, par phényle, par thiényle ou par phényle substitué;

$R^3$ est choisi parmi hydrogène, formyle, cycloalkyle ayant 3 à 8 atomes de carbone, alkényle ayant 2 à 8 atomes de carbone, alkényle ayant 3 à 8 atomes de carbone et substitué par jusqu'à 6 fluors, alkynyle ayant de 3 à 8 atomes de carbone, cycloalkényle ayant de 5 à 8 atomes de carbone, acyloxyalkyle ayant 2 à 12

atomes de carbone, phényle, phényle substitué, alkyle inférieur, X—$R^6$, -alkyl—Y—$C_pH_{2p+1}$ où la portion alkyle a 1 à 6 atomes de carbone, —$(CH_2)_nCONR^7R^8$, —$(CH_2)_mCO$—$OR^9$, et alkyle inférieur substitué par hydroxy, par sulfhydryle, par cyano, par amino, par halo, par cycloalkyle ayant de 3 à 8 atomes de carbone, par phényle, par thiényle ou par phényle substitué;

où X est O, NH ou S;

$R^6$ est phényle, phényle substitué, alkyle inférieur ou alkyle inférieur substitué par cycloalkyle ayant de 3 à 8 atomes de carbone ou par phényle;

p est un nombre entier de 0 à 4;

Y représente CO, O, S, $S^+$—$O^-$, $SO_2$ ou $=NC_rH_{2r+1}$ où r est un nombre entier de 0 à 4, à condition que p soit un nombre entier de 1 à 4 quand Y est $S^+$—$O^-$ ou $SO_2$;

$R^7$ et $R^8$ sont indépendamment hydrogène ou alkyle inférieur;

n est un nombre entier de 0 à 6;

$R^9$ est hydrogène ou alkyle inférieur;

et m est un nombre entier de 0 à 6;

$R^4$ est choisi parmi hydrogène, phényle, thiényle, pyridyle, phényle substitué, alkyle inférieur et alkyle inférieur substitué par cycloalkyle ayant de 3 à 8 atomes de carbone, par phényle, par thiényle ou par phényle substitué; et

$R^5$ est choisi parmi hydrogène et alkyle ayant de 1 à 4 atomes de carbone;

le terme "substitué", relativement aux groupes phényle, pyridyle et thiényle, signifie de tels groupes substitués par 1 à 3 substituants indépendamment choisis parmi halogène, trifluorométhyle, $CONH_2$, —$CO_2H$, hydroxy, —$S(O)_aR^{10}$ où $R^{10}$ est alkyle inférieur et a est 0, 1 ou 2, —$OR^{11}$ où $R^{11}$ est alkyle inférieur, ou $COR^{12}$ où $R^{12}$ est alkyle inférieur ou alcoxy ayant de 1 à 6 atomes de carbone;

et le terme "inférieur" relativement aux groupes alkyles indique de tels groupes ayant de 1 à 6 atomes de carbone.

2. Composé selon la revendication 1 ayant la formule de structure I et ses sels acceptables en pharmacie, où

$R^1$ et $R^2$ sont indépendamment choisis parmi alkyle ayant de 1 à 4 atomes de carbone;

$R^3$ est hydrogène, alkényle ayant de 2 à 8 atomes de carbone qui peut être substitué par jusqu'à 6 fluors, alkynyle ayant de 3 à 8 atomes de carbone, cycloalkényle ayant de 5 à 8 atomes de carbone, alkyle inférieur ou alkyle inférieur substitué par phényle;

$R^4$ est alkyle inférieur qui est substitué par phényle, par thiényle ou par phényle substituè;

et $R^5$ est hydrogène.

3. Composé selon la revendication 2 où $R^1$ et $R^2$ sont alkyle ayant 1 à 3 atomes de carbone, de préférence tous deux méthyle.

4. Composé selon l'une quelconque des revendications 1 à 3 où $R^3$ est hydrogène, alkényle ayant de 3 à 8 atomes de carbone qui peut être substitué avec jusqu'à 6 fluors, alkynyle ayant 3 à 8 atomes de carbone, cycloalkényle ayant 5 à 8 atomes de carbone, alkyle inférieur ou alkyle inférieur substitué par phényle.

5. Composé selon la revendication 4 où $R^3$ est hydrogène, méthyle, n-propyle, 2-propynyle, allyle, trans-2-buténylе, 2-cyclohexényle, —$CH_2CH=C(CH_3)_2$, —$CH_2CH=C(CF_3)CH_3$, —$CH_2CH=C(CF_3)_2$ ou benzyle.

6. Composé selon l'une quelconque des revendications 1 à 5 où $R^4$ est benzyle ou benzyle substitué, où "substitué" a la signification donnée à la revendication 1 relativement au phényle, en particulier p-fluorobenzyle ou 2-thiénylméthyle.

7. Composé selon la revendication 1 choisi parmi:

9-benzyl-2,3-dihydro-1,3-diméthyl-6-hydroxy-7-propyl-pyrimido[2,1-f]purine-4,8(1*H*,9*H*)-dione;

9-benzyl-2,3-dihydro-1,3-diméthyl-6-hydroxy-pyrimido[2,1-f]purine-4,8(1*H*,9*H*)-dione;

9-benzyl-2,3-dihydro-1,3-diméthyl-6-hydroxy-7-méthyl-pyrimido[2,1-f]purine-4,8(1*H*,9*H*)-dione;

7,9-dibenzyl-2,3-dihydro-1,3-diméthyl-6-hydroxy-pyrimido[2,1-f]purine-4,8(1*H*,9*H*)-dione;

9-benzyl-2,3-dihydro-1,3-diméthyl-7-formyl-6-hydroxy-pyrimido[2,1-f]purine-4,8(1*H*,9*H*)-dione;

9-benzyl-2,3-dihydro-1,3-diméthyl-7-(2-propynyl)-6-hydroxy-pyrimido[2,1-f]purine-4,8(1*H*,9*H*)-dione;

9-benzyl-7-(trans-2-buténуl)-2,3-dihydro-1,3-diméthyl-6-hydroxy-pyrimido[2,1-f]purine-4,8(1*H*,9*H*)-dione;

9-benzyl-7-(3-cyclohexényl)-2,3-dihydro-1,3-diméthyl-6-hydroxy-pyrimido[2,1-f]purine-4,8(1*H*,9*H*)-dione;

9-benzyl-2,3-dihydro-1,3-diméthyl-7-(éthoxycarbonylméthyl)-6-hydroxy-pyrimido[2,1-f]purine-4,8(1*H*,9*H*)-dione;

et en particulier

9-benzyl-2,3-dihydro-1,3-diméthyl-6-hydroxy-7-(3-méthyl-2-buténуl)-pyrimido[2,1-f]purine-4,8(1*H*,9*H*)-dione;

et leurs sels de sodium.

8. Composé selon la revendication 1 choisi parmi:

2,3-dihydro-1,3-diméthyl-9-(4-fluorobenzyl)-6-hydroxy-7-propyl-pyrimido[2,1-f]purine-4,8(1*H*,9*H*)-dione;

2,3-dihydro-1,3-diméthyl-9-(4-fluorobenzyl)-6-hydroxy-7-(3-méthyl-2-buténуl)-pyrimido[2,1-f]purine-4,8(1*H*,9*H*)-dione;

2,3-dihydro-1,3-diméthyl-9-(4-fluorobenzyl)-6-hydroxy-pyrimido[2,1-f]purine-4,8(1*H*,9*H*)-dione;

2,3-dihydro-1,3-diméthyl-9-(4-fluorobenzyl)-6-hydroxy-7-(2-propényl)-pyrimido[2,1-f]purine-4,8(1*H*,9*H*)-dione;

2,3-dihydro-1,3-diméthyl-9-(4-méthoxybenzyl)-6-hydroxy-7-(3-méthyl-2-buténył)-pyrimido[2,1-f]purine-4,8(1*H*,9*H*)-dione;

et leurs sels de sodium.

9. Composé selon la revendication 1, c'est-à-dire

2,3-dihydro-1,3-diméthyl-9-(2-thiénylméthyl)-6-hydroxy-7-propyl-pyrimido[2,1-f]purine-4,8(1*H*,9*H*)-dione;

et son sel de sodium.

10. Procédé de préparation de composés de la formule I définis à la revendication 1 qui comprend la réduction d'un composé de formule IV

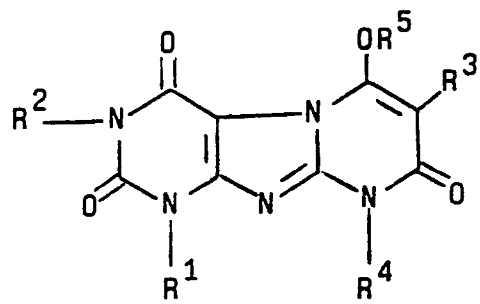

IV

ou son dérivé silylé formé avec le 1,1,1,3,3,3-hexaméthyldisilazane, où $R^1$, $R^2$, $R^3$, $R^4$ et $R^5$ sont tels que définis à la revendication 1, avec un agent réducteur qui est capable de réduire sélectivement le groupe 2-oxo;

et, si on le souhaite, en soumettant le composé résultant de la formule I à une ou plusieurs des étapes facultatives suivantes de finissage en tout ordre approprié:

a) réaction d'une composé de la formule I ou $R^5$ est hydrogène avec un diazoalcane ayant jusqu'à 6 atomes de carbone pour donner un composé de la formule I ou $R^5$ est un groupe alkyle ayant jusqu'à 6 atomes de carbone;

b) réaction d'un composé de la formule I où $R^3$ et $R^4$ sont hydrogène avec un électrophile activé $R^3Q$ ou $R^3$ est tel que défini ici et Q est un groupe partant efficace, en particulier un atome d'halogène, en particulier du chlore ou du brome;

c) isolement du produit final de la formule I tel quel ou sous la forme de son sel acceptable en pharmacie tel que défini ci-dessus.

11. Procédé selon la revendication 10 où l'agent réducteur est le borohydrure de lithium dans un éther solvant inerte ou l'hydrure de bis(2-méthoxyéthoxy)-aluminium sodium dans un solvant organique inerte comprenant un éther ou le borohydrure de tétrabutylammonium dans un éther solvant inerte.

12. Procédé selon la revendication 10 ou la revendication 11 où, à l'étape b), l'électrophile activé de la formule $R^3Q$ est un 3-halo-alcène, un 3-halo-alcyne, un a-halo ester, un halogénure de benzyle ou un a-halo-acétonitrile, en présence d'une base forte et d'un solvant organique inerte ou bien un sel de tétraalkyl ammonium dans un mélange d'un solvant organique inerte en conditions de transfert de phase.

13. Procédé de préparation d'un composé de formule I défini à la revendication 1 qui comprend la réaction d'un composé ayant le formule Ia

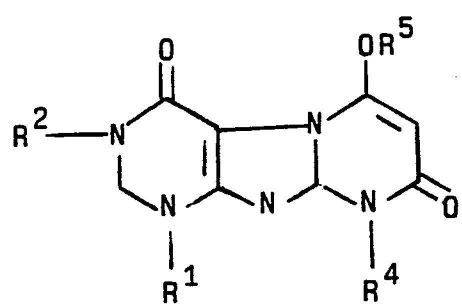

Ia

où $R^1$, $R^2$, $R^4$ et $R^5$ sont tels que définis à la revendication 1 avec un électrophile activé capable d'introduire le groupe $R^3$.

14. Procédé de la revendication 13 où l'électrophile activé est un 3-halo-alcène, un 3-halo-alcyne, un a-halo ester, de l'halogénure de benzyle ou un a-halo-acétonitrile.

15. Composition pharmaceutique qui comprend un composé de formule I défini à la revendication 1 ou son tautomère ou sel acceptable en pharmacie, en combinaison avec un véhicule acceptable en pharmacie.

16. Utilisation d'un composé de la formule I défini à la revendication 1 ou son tautomère ou sel acceptable en pharmacie pour la préparation de compositions pharmaceutiques utiles pour le traitement d'une inflammation chez un mammifère.